# EUROPEAN PATENT APPLICATION

(11) **EP 2 458 014 A1**
(43) Date of publication of application: **30.05.2012**
(21) Application number: 10175599.9
(22) Date of filing: 07.09.2010
(51) Int. Cl.: C12Q 1/68

(54) **Prognostic markers for acute myeloid leukemia (AML)**

(71) Applicant: Rijksuniversiteit Groningen, 9712 CP Groningen (NL); Academisch Ziekenhuis Groningen, 9713 GZ Groningen (NL)
(72) Inventor: The designation of the inventor has not yet been filed
(74) Representative: Jansen, Cornelis Marinus

(57) **Abstract**

The invention relates generally to the field of cancer and more in particular to cancer prognostics and treatment protocols. Provided is a method for predicting the disease prognosis of a subject suffering from acute myeloid leukemia (AML), said method comprising a. obtaining an isolated sample from a subject; b. screening for a simultaneous aberrant expression level of two or more biomarkers in the sample, the biomarkers being selected from the group consisting of ankyrin repeat domain 28 (ANKRD28), guanine nucleotide binding protein alpha 15 (GNA15), UDP-glucose pyrophosphorylase (UGP2) and c. scoring the expression level as being aberrant when the expression level detected is above a certain threshold coefficient; wherein the detection threshold coefficient is determined by comparing the expression levels of the samples obtained from the subject to values in a reference database of sample phenotypes obtained from healthy subjects, wherein the presence of an aberrant expression level of two or more markers is indicative of a poor disease prognosis in the subject. Also provided are predictor sets and diagnostic kits.

## Description

The present invention relates generally to the field of cancer. In particular, the present invention relates to cancer prognostics and treatment protocols. The invention can be applied in the biomedical sector, its subject being a method for predicting the disease prognosis of a subject suffering from acute myeloid leukemia (AML). An additional aspect concerns reagents and a kit for the diagnosis/prognosis of the aforesaid disorder and its use.

AML is clinically, cytogenetically and molecularly a heterogeneous disease. The heterogeneity within AML makes it challenging to classify the disease and determine prognosis. Currently, the WHO classification is used for defining separate risk groups based on cytogenetic and molecular abnormalities.¹ However, especially in the intermediate cytogenetic risk group, which represents the largest AML subgroup (±60%), treatment outcome varies considerably. At the moment, within the intermediate cytogenetic risk group subgroups are recognized based on mutations of the nucleophosmin 1 (*NPM1*) and fms-related tyrosine kinase 3 (*FLT3*) gene.^{2,3} This categorization is not only of relevance for risk stratification upfront treatment but also for the treatment applied. Patients with *NPM1*/*FLT3*-ITD mutations are treated only with chemotherapy, while the optimal treatment for patients belonging to the *NPM1* wild type subgroup consists of chemotherapy and allogeneic stem cell transplantation. Therefore, identification of novel molecular markers might be helpful for further risk stratification. In recent years, a number of gene expression profiling (GEP) studies has been performed in order to improve the identification of known cytogenetic subgroups and to recognize new clusters of AML patients with distinct gene-expression signatures.⁴⁻⁹ However, thus far a wide variability has been recognized in the composition of gene sets that define the separate prognostic subgroups. In part this variability might be due to interfering transcriptional background as a result of the use of heterogeneous cell populations. Most AML GEP studies are performed with the total AML mononuclear cell (MNC) fraction. Since cell lineage and differentiation stages affect gene-expression based clustering,^{4, 5, 8} the differential expression of genes associated with the differentiation stage might obscure more basic gene expression information related to tumor initiation and maintenance. With the availability of the mircroarray technique, GEP has been applied to derive prognostic signatures for AML that would identify subsets of patients with differing outcomes. In normal karyotype AML patients, Bullinger et al. were able to distinguish two different prognostically relevant clusters using 133 probe sets.⁶ More recently, an additional study in normal karyotype AML revealed a gene signature of 86 probe sets correlating significantly with OS in 163 patients, which could be validated and confirmed in an independent cohort of 79 normal karyotype AML patients.³² A limitation in applying these results to clinical practice is the number of genes that have to be studied.

There are other parameters that predict prognosis in normal karyotype AML such as age, white blood cell count, and the presence of FLT3-ITD, mutations of NPM1 and C/EBPα genes but these parameters are not yet sufficient to reliably predict prognosis so that the appropriate treatment modality can be chosen. Thus, although current prognostic criteria and molecular markers provide some guidance in predicting patient outcome and selecting appropriate course of treatment, a significant need exists for more powerful and independent prognostic factors that can guide treatment decisions, especially for the large subgroup of patients presenting with normal karyotype AML. Such a method should specifically distinguish AML patients with a poor prognosis from those with a good prognosis and permit the identification of high-risk patients who are likely to need additional (adjuvant) therapy, such as bone marrow transplantation.

The present invention aims at overcoming at least one of the above deficiencies by providing clinically relevant prognostic tools useful for risk stratification of AML patients, as well as identifying patients that would benefit from a given therapeutic regiment, for example bone marrow transplantation. The present inventors hypothesized that profiling of more purified cell populations instead of total MNC fractions could enhance the feasibility of GEP in identifying novel prognostic markers. More specifically, it was surprisingly found that CD34+ enriched AML cell fractions are of particular interest for identify prognostic markers. In the present study, GEP was performed on purified CD34+ AML cells and the results were compared with normal CD34+ cells. This allowed the identification of a top 50 list of genes which were differentially expressed in AML CD34+ versus normal CD34+ cells and versus AML CD34- cells.

By using the more purified AML CD34+ population and a large cohort of normal bone marrow samples the present inventors were able to identify a relatively small gene set with strong prognostic significance. A single PCR measurement of these genes can be easily incorporated in routine diagnostic measurements. In conclusion, we identified a set of genes of which the expression was showed to be independently prognostic relevant in normal karyotype AML. This gene set can be of help in classifying the subgroup of patients presenting with a normal karyotype AML and improve treatment decisions.

Accordingly, in one embodiment the invention relates to a method for predicting the disease prognosis of a subject suffering from acute myeloid leukemia (AML), said method comprising:
a. obtaining an isolated sample from a subject;
b. screening for an aberrant expression level of one or more biomarkers in the sample, the biomarkers being selected from Table 2 and
c. scoring the expression level as being aberrant when the expression level detected is above a certain threshold coefficient; wherein the detection threshold coefficient is determined by comparing the expression levels of the samples obtained from the subject to values in a reference database of sample phenotypes obtained from healthy subjects, wherein the presence of an aberrant expression level of one or more, preferably two or more, markers is indicative of a poor disease prognosis in the subject.

As used herein, a subject suffering from AML is a human subject of any age. For example, it is an infant, a juvenile, a teenager, an adult or an elderly person. In one embodiment, the subject suffers from normal karyotype (NK) AML. By "sample" is intended any sampling of cells, tissues, or bodily fluids in which expression of a biomarker can be detected. Examples of such body samples include blood, lymph, urine, bodily fluids, biopsies, and smears. Body samples may be obtained from a patient by a variety of techniques including, for example, by scraping or swabbing an area or by using a needle to aspirate bodily fluids. Methods for collecting various body samples are well known in the art. Preferred samples are those which can be obtained in a non-invasive or relatively non-invasive manner. Included are blood samples and bone marrow samples. In the method of the present invention, it is preferred that the biological sample obtained from the subject is whole blood, blood serum or plasma.

A "biomarker" is any gene or protein whose level of expression in a biological sample is altered compared to that of a pre-determined level. The predetermined level can be a level found in a biological sample from a normal or healthy subject.

The screening for aberrant expression levels of one or more biomarkers can be performed by methods known in the art, for example using PCR-based technology. Multiple means for such analysis are available, including detection of expression within an assay for global, or near global, gene expression in a sample (e.g. as part of a gene expression profiling analysis such as on a microarray) or by specific detection, such as quantitative PCR (Q-PCR), or real time quantitative PCR, Western blot or any other assay well known to one skilled in the art.

As used herein, the term "poor disease prognosis" comprises disease recurrence, relapse, poor overall survival prognosis, poor disease free survival, or a combination thereof.

Any of the listed biomarkers may be used individually to assess the disease prognosis of a subject. The selection of one or more biomarkers whose aberrant expression level is most pronounced (e.g. at least 1.5 or at least 2-fold compared to controls) is generally preferred. In addition, it will be understood that the prognostic value of a method of the invention assessment will increase with the number of individual biomarkers investigated. Therefore, it is advantageous to screen for the simultaneous aberrant expression level of two or more, preferably three or more, like 4 or 5, or even up to 10 different biomarkers selected from those listed in Table 2.

Interestingly, for at least three of these genes (ANKRD28, GNA15 and UGP2) a high transcript level was associated with a significant poorer OS in normal karyotype AML patients, as validated in two independent and large cohorts of normal karyotype AML patients. Importantly, the prognostic value of the continuous transcript levels of ANKRD28, GNA15 and UGP2 was shown to be independent from the well known risk factors FLT3-ITD, NPM1c+ and CEBPA mutation status. Therefore, it may be preferred to include a specific combination of at least two or three biomarkers, e.g. ANKRD28 and GNA15, ANKRD28 and UGP-2, GNA15 and UGP-2, or ANKRD28, GNA15 and UGP-2. In a specific aspect the invention provides a method for predicting the disease prognosis of a subject suffering from acute myeloid leukemia (AML), said method comprising:
a. obtaining an isolated sample from a subject;
b. screening for a simultaneous aberrant expression level of two or more biomarkers in the sample, the biomarkers being selected from the group consisting of ankyrin repeat domain 28 (ANKRD28), guanine nucleotide binding protein alpha 15 (GNA15), UDP-glucose pyrophosphorylase (UGP2) and
c. scoring the expression level as being aberrant when the expression level detected is above a certain threshold coefficient; wherein the detection threshold coefficient is determined by comparing the expression levels of the samples obtained from the subject to values in a reference database of sample phenotypes obtained from healthy subjects, wherein the presence of an aberrant expression level of two or more markers is indicative of a poor disease prognosis in the subject. Preferably, in a method of the invention the aberrant expression level is an increased cumulative expression of two or more biomarkers in the sample, the biomarkers being selected from the group consisting of ANKRD28, GNA15 and UGP2. Very good results can be obtained when screening for a simultaneous increased expression level of ANKRD28, GNA15 and UGP2.

The invention also provides a predictor set use in a method according to the invention comprising one or more of the predictor biomarker genes of the invention as shown in Table 2. In one embodiment, the predictor set comprises a collection of two or more isolated binding partners which bind selectively to the products of at least two biomarkers, wherein the biomarkers are selected from the group consisting of human ankyrin repeat domain 28 (ANKRD28), guanine nucleotide binding protein alpha 15 (GNA15) and UDP-glucose pyrophosphorylase (UGP2). For example, the predictor set comprises or consists of a collection of distinct isolated binding partners which bind selectively to the products of the biomarkers ANKRD28, GNA15 and UGP2. As used herein, "distinct isolated binding partners" refers to a set of different entities, each entity being capable of recognizing and binding to its biomarker counterpart. The chemical nature of the binding partner can vary and may depend on the chemical nature of the biomarker to be detected (e.g. protein or nucleic acid). In one embodiment, both the binding partners and the biomarker products are proteins.

A preferred agent for detecting and quantifying an AML prognostic biomarker disclosed herein at the protein level is an antibody able to bind specifically to this protein, preferably an antibody with a detectable label. Antibodies can be polyclonal, or more preferably, monoclonal. As used herein, the term encompasses not only intact polyclonal or monoclonal antibodies, but also fragments thereof (such as Fab, Fab', F(ab')2, Fv), single chain (ScFv), mutants thereof, fusion proteins comprising an antibody portion, humanized antibodies, chimeric antibodies, diabodies linear antibodies, single chain antibodies, multispecific antibodies (e.g., bispecific antibodies) and any other modified configuration of the immunoglobulin molecule that comprises an antigen recognition site of the required specificity. An antibody includes an antibody of any class, such as IgG, IgA, or IgM (or sub-class thereof), and the antibody need not be of any particular class. The antibody may be used in an immunoassay method or kit of the invention, for instance ELISA, antibody coated tube test, lateral-flow test or biosensor.

In another embodiment, the binding partners are isolated polynucleotides which bind selectively to the RNA products of at least two biomarkers. Exemplary polynucleotide binding partners include single or double stranded RNA and single or double stranded DNA. A preferred agent for detecting and quantifying mRNA or cDNA encoding a biomarker of the invention is a labeled nucleic acid probe or primers able to hybridize this mRNA or cDNA. The nucleic acid probe can be an oligonucleotide of at least 10, 15, 30, 50 or 100 nucleotides in length and sufficient to specifically hybridize under stringent conditions to the mRNA or cDNA. The nucleic acid primer can be an oligonucleotide of at least 10, 15 or 20 nucleotides in length and sufficient to specifically hybridize under stringent conditions to the mRNA or cDNA, or complementary sequence thereof. In certain embodiments of the method of the present invention, the determination of one or more AML biomarker transcripts involves the use of a probe/primer in a polymerase chain reaction (PCR), such as anchor PCR or RACE PCR, or, alternatively, in a ligation chain reaction (LCR) (see, e.g., Landegran et al., 1988, Science 241 :23- 1080; and Nakazawa et al., 1994, Proc. Natl. Acad. Sci. USA, 91 :360-364), or alternatively quantitative real time RT-PCR This method can include the steps of collecting a sample of cells from a patient, isolating nucleic acid (e.g. mRNA) from the cells of the sample, optionally transforming mRNA into corresponding cDNA, contacting the nucleic acid sample with one or more primers which specifically hybridize to the biomarker mRNA or their corresponding cDNA under conditions such that hybridization and amplification of the biomarker mRNA or cDNA occurs, and quantifying the presence of the amplification products. It is anticipated that PCR and/or LCR may be desirable to use as an amplification step in conjunction with any of the techniques used for quantifying nucleic acid detecting.

The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.
For example, *in vitro* techniques for detection of mRNA include Northern hybridizations and in situ hybridizations. *In vitro* techniques for detection of the biomarker protein include enzyme linked immunosorbent assays (ELISAs), Western blots, immunoprecipitations and immunofluorescence. *In vitro* techniques for detection of a biomarker cDNA include Southern hybridizations.

It may be advantageous to bind or immobilize the binding partners being bound to a solid support. The solid support may comprise components of glass, silica, cellulose, cellulose acetate, nitrocellulose, nylon, polyester, polyethersulfone, polyolefin, or polyvinylidene fluoride, or combinations thereof. The solid support can be an array surface, a (magnetic) bead or any other type of carrier. In one embodiment, the binding partners are bound to the solid support in an addressable location. In a specific aspect, one or more reagents necessary to the detection or the quantification of the biomarker(s) may be immobilized onto solid support, such as biochips to form a two- dimension array, for example, a 9 mm x 9 mm array, 12 mm x 12 mm array, and 15 mm x 15 mm array. One or more arrays may be arranged on one biochip, and one or more samples can be tested using one biochip. In some embodiments, the solid support of the biochip comprises a surface selected from the group consisting of a ceramic, a glass, a silica, a quartz, a nylon, a plastic, a polystyrene, a nitrocellulose, and a metal. This type of support and method using biochip (protein or nucleic acid biochip) are well known by the skilled man to perform diagnosing tests.

Are also comprised in the present invention the kits of the present invention which are suitable for performing the method of the invention. Provided is a diagnostic kit comprising a predictor set described herein above, and instructions for use to predict the disease prognosis of a subject suffering from acute myeloid leukemia (AML), preferably NK-AML. In one embodiment, the kit comprises : a) a binding partner directed specifically against ANKRD28; and b) an binding partner directed specifically against at least one of the polypeptide selected from the group consisting of GNA15 and UGP2. In another embodiment, the kit comprises : a) a binding partner directed specifically against GNA15 and b) an binding partner directed specifically against at least one of the polypeptide selected from the group consisting of ANKRD28 and UGP2. In yet another embodiment, the kit comprises : a) a binding partner directed specifically against UGP2; and b) an binding partner directed specifically against at least one of the polypeptide selected from the group consisting of GNA15 and ANKRD28. Preferably, the kit comprises : a) a binding partner directed specifically against ANKRD28; b) an binding partner directed specifically against GNA15 and c) an binding partner directed specifically against UGP2.

For example, provided is a kit comprising : a) an antibody directed specifically against ANKRD28; and b) an antibody directed specifically against at least one of the polypeptide selected from the group consisting of GNA15 and UGP2. In another embodiment, the kit comprises : a) an antibody directed specifically against GNA15 and b) an antibody directed specifically against at least one of the polypeptide selected from the group consisting of ANKRD28 and UGP2. In yet another embodiment, the kit comprises : a) an antibody directed specifically against UGP2; and b) an antibody directed specifically against at least one of the polypeptide selected from the group consisting of GNA15 and ANKRD28. Preferably, the kit comprises : a) an antibody directed specifically against ANKRD28; b) an antibody directed specifically against GNA15 and c) an antibody directed specifically against UGP2.

Still further, there is provided a kit comprising : a) a set of primers capable of amplifying specifically the ANKRD28 RNA or cDNA; and b) a set of primers capable of amplifying specifically the RNA or cDNA from the group consisting of GNA15 and UGP2 RNA or cDNA. Still further, there is provided a kit comprising : a) a set of primers capable of amplifying specifically the GNA15 RNA or cDNA; and b) a set of primers capable of amplifying specifically the RNA or cDNA from the group consisting of ANKRD28 and UGP2 RNA or cDNA. Still further, there is provided a kit comprising : a) a set of primers capable of amplifying specifically the UGP2 RNA or cDNA; and b) a set of primers capable of amplifying specifically the RNA or cDNA from the group consisting of GNA15 and ANKRD28 RNA or cDNA.
Also provided is a kit comprising : a) a nucleic probe capable of hybridising specifically with the ANKRD28 RNA; and b) a nucleic probe capable of hybridising specifically with at least one of the RNA selected from the group consisting of GNA15 and UGP2 RNA.

As will be understood, the present invention is also directed to the use of the expression of the one or more genes of Table 2 as a biomarker, preferably as a serum (or plasma) or cellular biomarker, for the predicting the disease prognosis of an AML patient, particularly of an NK-AML patient. The use of UGP2, GNA15 and/or ANKRD28 is preferred.

A further aspect relates to a method for choosing the treatment modality for an individual subject suffering from AML, comprising predicting the disease prognosis of said subject according to a method of the invention, for instance using a predictor set and/or using a kit as described herein above, and further comprising selecting a treatment regimen based on the disease prognosis. Selecting a treatment regimen may comprise determining whether the therapy should include or exclude a (autologous or allogenic) bone marrow transplantation.

Also within the scope of the invention is a method for identifying a biomarker useful for predicting the disease prognosis of an AML subject, comprising the steps of:
a. providing a first population of mononuclear cells derived from subjects known to suffer from AML
b. enriching said population for CD34 positive mononuclear cells, for instance using anti-CD34 magnetic beads,
c. performing gene expression profiling on RNA obtained from said enriched population, preferably using microarray technology,
d. comparing the gene expression profile with a profile obtained from CD34 enriched population of healthy subjects
e. determining which genes are differentially expressed between the AML
f. performing univariate Cox regression analyses between the differentially expressed genes and disease progression in a population of de novo cytogenetically normal AML
g. identifying the gene(s) showing a significant (P<0.05) association with poor disease progression, in particular wherein poor disease progression is poor overall survival or poor event free survival.

### EXPERIMENTAL SECTION

### Methods

### Patient material

GEP was performed on blast cells of 46 patients with AML from a single center (University Medical Center Groningen). The diagnosis of AML was based on cytological examination, immunophenotyping and cytogenetic analysis of blood and bone marrow. AML blasts were collected from peripheral blood (PB) or bone marrow (BM) after achieving informed consent. The study was approved by the Medical Ethical Committee of the UMCG. MNC were isolated by density gradient centrifugation using lymphoprep (PAA, C61be, Germany). The cells were cryopreserved in aliquots of 20 to 50 x 10⁶ cells/ml in 10% dimethylsulfoxide (Sigma, St Louis, MO, USA) and 10% fetal calf serum (Life Technologies, Breda, The Netherlands). After thawing, CD34⁺ and CD34⁻ AML cells were selected by MoFLo sorting (Dako Cytomation, Carpinteria, CA, USA) using a CD34 PE-labeled antibody (Clone 8G12, BD Biosciences, San Jose, California, USA). Cytogenetic risk group distinction (favorable, intermediate, and unfavorable) according to current HOVON/SAKK protocols is provided in Table 1.^{22;23} Potential donors for allogeneic bone marrow transplantation and patients who underwent elective total hip replacement served as normal controls (n=31) after providing informed consent. From the normal bone marrow aspirates the MNC fraction was isolated and CD34-enriched fractions were obtained using anti-CD34 magnetic beads (Clone QBEND/10, Miltenyi Biotec, Auburn, CA, USA).

### Analysis of mutations in NPM1 and FLT3

Detection of *NPM1* mutations was performed by immunohistochemical staining on deparaffinized bone marrow biopsies using a Benchmark XT (Ventana Medical Systems S.A., USA). The antigens were retrieved with EDTA buffer (pH 8.5) and endogenous peroxidase was blocked with H₂O₂. Slides were incubated with 1:50 diluted supernatant of the biotinylated anti-NPM1 antibody (kindly provided by Prof. Falini, Perugia, Italy). Mutational analysis of ITD within the JM domain of the *FLT3* gene was performed with RT-PCR as previously described.²⁴

### Gene expression profiling

Total RNA was isolated using the RNeasy mini kit from Qiagen (Venlo, The Netherlands) according to the manufacturer's recommendations. RNA quality was examined using the Agilent 2100 Bioanalyzer (Agilent Technologies, Waldbronn, Germany). Genome-wide expression analysis was performed on Illumina (Illumina, Inc., San Diego, CA) BeadChip Arrays Sentrix Human-6 (46k probesets). Typically, 0.5-1 µg of mRNA was used in labeling reactions and hybridization with the arrays was performed according to the manufacturer's instructions. The RMA method in R version 2.4.0 was used to compute probe sets summaries.²⁵ Quantile normalization was applied to log₂-transformed intensities.²⁶ Principal component analysis (PCA) was performed for quality control. The most significant principal component for a gene expression data matrix is frequently a constant pattern, which dominates the data.^{27;28} So, the first principal component explaining the largest part of the variation could be considered variation that the arrays have in common.^{29;30} Correlation with the first principal component was calculated for each individual array (factor loading). Factor loadings of the first principal component for an individual array can be seen as a quality index, as arrays of lesser quality would have lower or distinctly different correlations than arrays of high quality. Samples with a factor loading with the first principal components of less than 2 times the standard deviation from the mean were excluded as their hybridizations were considered to be of low quality.^{29;30} To perform further analyses two publicly available cytogenetically normal AML data sets (GSE16891 [NCBI GEO] ; n = 218)^{5;31} and (GSE12417 [NCBI GEO] ; n = 163)³² were utilized. The selected data sets provided us with clinically-annotated gene expression values from Affymetrix Human Genome U133A or U95 gene chip array (Affymetrix, Santa Clara, CA).

### Class comparison

Class comparison was performed using the software package Biometric Research Branch ArrayTools (BRB ArrayTools) version 3.6.0, developed by the Biometric Research Branch of the US National Cancer Institute (http://linus.nci.nih.gov/BRB-ArrayTools.html). As the CD34⁺ and CD34⁻ AML samples were paired samples (n=44), a paired t-test was used to identify differentially expressed genes. For the comparison CD34⁺ AML (n=46) versus CD34⁺ NBM (n=31), as well as CD34⁻ AML (n=44) versus CD34⁺ NBM (n=31) a random variance t-test was used. Differential expression was considered significant at *P* < 0.0001. Average linkage hierarchical clustering with the centered correlation distance metric was performed using Cluster 3.0 and TreeView software.³³

### Statistical analyses

Statistical analyses were performed with SPSS software, release 16.0. Actuarial probabilities of overall survival (OS) (with death due to any cause) as well as event-free survival (EFS, with failure in case of no complete remission [CR1] or relapse or death) were estimated according to the Kaplan-Meier method. For quantitative parameters overall differences between the cohorts were evaluated using an F-test (or Student-t in case of two groups) for normally distributed variables or a Kruskal-Wallis test (or Mann-Whitney-U test in case of two groups) for skewed distributed variables. For qualitative parameters, overall group differences were evaluated using a Chi-square test (or Fisher exact in 2x2 setting). Correlations were calculated with the Spearman rank correlation coefficient (rho). The association between transcript levels of the top 50 CD34⁺ AML specific genes and OS and EFS was tested in univariate Cox models. Cox regression analysis was applied to determine the association of *ANKRD28, GNA15* and *UGP2* and OS/EFS with adjustment for known disease-related risk factors such as *FLT3*-ITD, *NPM1c*+ and *CEBPA* mutations. Of note, when multiple probe sets representing the same gene were present on the Affymetrix arrays, probes were averaged. All tests were 2 tailed, and a *P* < .05 was considered statistically significant.

### Results

Samples were available from 46 AML patients, of which 44 were paired CD34⁺ and CD34⁻ samples. Patient characteristics are provided in Table 1. With regard to the risk group 7% of the patients belonged to the good risk group, 63% to the intermediate risk group and 28% to the poor risk group. Mean percentage of sorted CD34⁺ cells within the AML mononuclear cell fractions was 28.9% (ranging 1-90%).

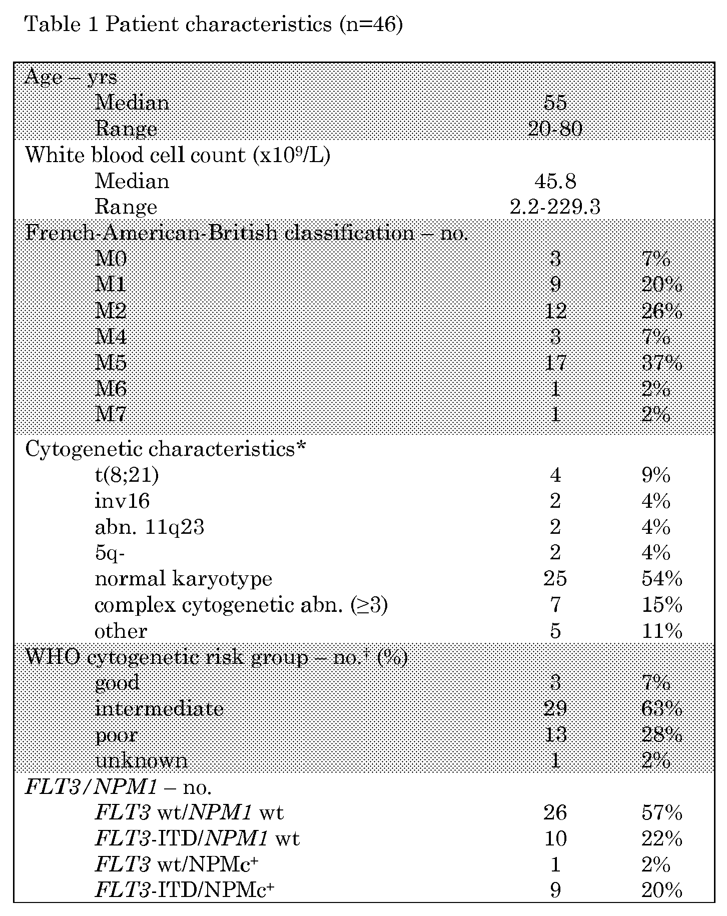

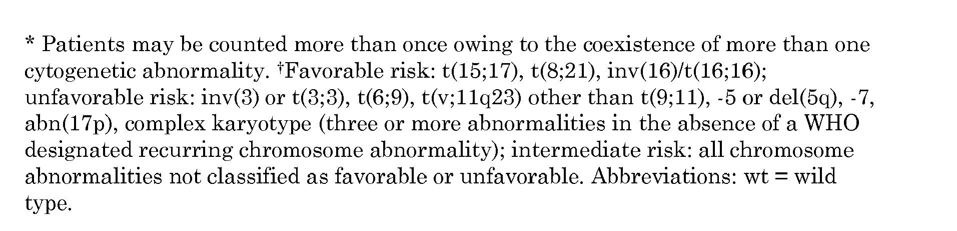

### Identification of CD34⁺ AML specific genes

Gene expression profiles were also compared between CD34⁺ AML cells versus CD34⁺ normal bone marrow (NBM) cells. This analysis revealed 3809 differentially expressed unique genes (2180 up and 1629 down in CD34⁺ AML). Furthermore, 3162 unique genes were found to be differentially expressed between CD34⁻ AMLs and CD34⁺ NBM samples (1717 up and 1445 down in CD34⁻ AML). 2133 genes were overlapping between both analyses, and thus differentially expressed between AML and NBM irrespective of CD34 status (hereinafter referred to as common AML specific genes). 1677 genes were specifically expressed in the AML CD34⁺ group versus CD34⁺ NBM, of which 1013 were significantly higher expressed in CD34⁺ AML cells compared to NBM cells. The top 50 of AML CD34⁺ specific genes is shown in Table 2.

**Table 2. Univariate cox regression analyses in n = 163 and n = 218 cytogenetically normal karyotype AMLs with regards to overall survival. HR indicates hazard ratio; CI, confidence interval,**

| | **163 cytogenetically normal karyotype AMLs** | | | | **218 cytogenetically normal karyotype AMLs** | | | |
|---|---|---|---|---|---|---|---|---|
| | | | **lower** | **upper** | | | **lower** | **upper** |
| | | | **95%** | **95%** | | | **95%** | **95%** |
| **Gene** | ***P*** | **HR** | **CI** | **CI** | ***P*** | **HR** | **CI** | **CI** |
| DUSP3 | 0.496 | 1.076 | 0.871 | 1.329 | 0.063 | 1.316 | 0.985 | 1.758 |
| CLIC1 | 0.152 | 1.419 | 0.879 | 2.291 | 0.025 | 1.911 | 1.084 | 3.37 |
| **ANKRD28** | **0.001** | **1.779** | **1.271** | **2.49** | **0.008** | **1.31** | **1.075** | **1.598** |
| DPAGT1 | 0.382 | 0.911 | 0.74 | 1.122 | 0.61 | 1.092 | 0.779 | 1.53 |
| ITGA5 | 0.113 | 1.24 | 0.951 | 1.616 | 0.182 | 1.248 | 0.902 | 1.726 |
| **GNA15** | **0.001** | **1.509** | **1.175** | **1.938** | **0.01** | **1.258** | **1.056** | **1.498** |
| FLT3 | 0.021 | 1.23 | 1.032 | 1.466 | 0.403 | 1.073 | 0.91 | 1.264 |
| C14ORF79 | 0.266 | 0.884 | 0.711 | 1.099 | 0.175 | 1.426 | 0.854 | 2.379 |
| GNPTAB | 0.001 | 2.559 | 1.501 | 4.362 | 0.049 | 1.625 | 1.001 | 2.639 |
| TFPI | 0.111 | 1.125 | 0.973 | 1.3 | 0.122 | 1.108 | 0.973 | 1.261 |
| BMP1 | 0.037 | 1.681 | 1.033 | 2.735 | 0.274 | 1.439 | 0.749 | 2.765 |
| PTTG1IP | 0.014 | 1.799 | 1.129 | 2.867 | 0.109 | 1.295 | 0.944 | 1.775 |
| HSBP1 | 0.585 | 0.895 | 0.603 | 1.33 | 0.09 | 1.35 | 0.955 | 1.908 |
| KDELR1 | 0.299 | 1.269 | 0.809 | 1.99 | 0.012 | 2.203 | 1.189 | 4.081 |
| CHKA | 0.038 | 0.8 | 0.648 | 0.988 | 0.975 | 0.995 | 0.739 | 1.341 |
| SMYD3 | 0.608 | 1.05 | 0.871 | 1.267 | 0.235 | 1.099 | 0.94 | 1.285 |
| TEC | 0.122 | 1.107 | 0.973 | 1.259 | 0.027 | 1.299 | 1.03 | 1.639 |
| MAPKAPK3 | 0.908 | 1.026 | 0.666 | 1.579 | 0.09 | 1.576 | 0.932 | 2.663 |
| PHKB | 0.934 | 0.987 | 0.717 | 1.358 | 0.269 | 1.221 | 0.857 | 1.741 |
| EFHC1 | 0.015 | 1.651 | 1.101 | 2.477 | 0.051 | 1.279 | 0.999 | 1.637 |
| LMAN2L | 0.551 | 1.034 | 0.927 | 1.153 | 0.1 | 1.486 | 0.927 | 2.382 |
| ELMO1 | 0.021 | 1.782 | 1.092 | 2.909 | 0.015 | 1.657 | 1.105 | 2.485 |
| PLEKHA9 | 0.623 | 1.052 | 0.859 | 1.289 | 0.882 | 0.975 | 0.701 | 1.357 |
| RAI1 | 0.04 | 1.54 | 1.02 | 2.324 | 0.719 | 1.095 | 0.668 | 1.796 |
| ITGA9 | 0.02 | 1.562 | 1.072 | 2.276 | 0.713 | 0.911 | 0.555 | 1.495 |
| SPATA20 | 0.677 | 1.058 | 0.811 | 1.38 | 0.633 | 1.062 | 0.831 | 1.357 |
| LEPRE1 | 0.938 | 1.01 | 0.784 | 1.302 | 0.744 | 1.059 | 0.751 | 1.492 |
| GRHPR | 0.677 | 1.081 | 0.748 | 1.563 | 0.168 | 1.269 | 0.904 | 1.782 |
| UBE2E1 | 0.012 | 1.85 | 1.143 | 2.995 | 0.249 | 1.346 | 0.813 | 2.228 |
| EEF2 | 0.879 | 0.939 | 0.419 | 2.107 | 0.528 | 1.295 | 0.58 | 2.89 |
| MRP63 | 0.011 | 1.938 | 1.162 | 3.234 | 0.082 | 1.474 | 0.952 | 2.28 |
| SDCCAG8 | 0.018 | 1.589 | 1.084 | 2.33 | 0.034 | 1.492 | 1.03 | 2.162 |
| CUTL1 | 0.017 | 1.895 | 1.123 | 3.199 | 0.412 | 1.141 | 0.832 | 1.566 |
| RAB6IP1 | 0.611 | 0.927 | 0.691 | 1.242 | 0.253 | 1.184 | 0.886 | 1.584 |
| CMAH | 0.084 | 1.381 | 0.957 | 1.993 | 0.052 | 1.269 | 0.998 | 1.613 |
| GCN5L2 | 0.327 | 1.188 | 0.842 | 1.677 | 0.248 | 1.172 | 0.896 | 1.533 |
| ZNF254 | 0.098 | 1.356 | 0.946 | 1.944 | 0.411 | 1.128 | 0.847 | 1.503 |
| LIAS | 0.737 | 1.024 | 0.892 | 1.175 | 0.42 | 1.104 | 0.868 | 1.404 |
| EIF2S3 | 0.092 | 1.478 | 0.938 | 2.328 | 0.415 | 1.169 | 0.804 | 1.699 |
| CEP290 | 0.508 | 1.08 | 0.859 | 1.359 | 0.243 | 1.191 | 0.888 | 1.598 |
| BCL2L2 | 0.025 | 1.878 | 1.081 | 3.262 | 0.751 | 1.07 | 0.704 | 1.627 |
| TPM4 | 0.01 | 1.58 | 1.116 | 2.237 | 0.146 | 1.233 | 0.93 | 1.636 |
| SYF2 | 0.015 | 1.914 | 1.136 | 3.226 | 0.695 | 1.077 | 0.743 | 1.562 |
| RPS29 | 0.037 | 1.609 | 1.028 | 2.517 | 0.483 | 1.143 | 0.787 | 1.659 |
| ZNF551 | 0.084 | 1.481 | 0.948 | 2.313 | 0.787 | 1.046 | 0.753 | 1.454 |
| XPNPEP1 | 0.051 | 2.038 | 0.996 | 4.171 | 0.803 | 1.071 | 0.624 | 1.839 |
| SNRPD3 | 0.542 | 1.097 | 0.814 | 1.479 | 0.244 | 1.337 | 0.82 | 2.181 |
| C1ORF149 | 0.007 | 2.95 | 1.352 | 6.436 | 0.1 | 1.79 | 0.895 | 3.582 |
| MRPS30 | 0.132 | 1.642 | 0.861 | 3.13 | 0.842 | 1.03 | 0.767 | 1.385 |
| **UGP2** | **0.001** | **2.626** | **1.507** | **4.578** | **0** | **2.247** | **1.44** | **3.505** |

### Prognostic value of top 50 AML CD34⁺ specific genes in normal karyotype AML

Next, we wondered whether this set of 50 CD34⁺ specific AML genes had prognostic significance. Therefore, univariate cox regression analyses were performed between the continuous transcript levels of these 50 CD34⁺ specific genes and OS in a large series of de novo cytogenetically normal karyotype AMLs³² (n = 163). These analyses revealed that 6 genes out of these 50 had predictive significance at a P-value ≤ .01 whereby higher expression levels correlated with poor survival rates.
Next, these findings were validated in an independent cohort of 218 cytogenetically normal karyotype AMLs⁵ (Table 2). Interestingly, higher transcript levels of three of the 50 CD34⁺ AML specific genes (i.e. ankyrin repeat domain 28 (*ANKRD28*), guanine nucleotide binding protein, alpha 15 (*GNA15*) and UDP-glucose pyrophosphorylase 2 (*UGP2*)) were associated with a significant poorer OS in both cohorts of normal karyotype AML at the significance of *P* < .01 (Table 2). For the cohort of 218 normal karyotype AMLs also event free survival (EFS) data were available for further analyses. A significant association between the continuous transcript levels of *ANKRD28, GNA15* and *UGP2* with unfavourable EFS was evident (*ANKRD28:* HR: 1.30, 95% CI: 1.07-1.56, *P* = .007; *GNA15*: HR: 1.23, 95% CI: 1.05-1.45, *P* = .012 and *UGP2:* HR: 2.04, 95% CI: 1.34-3.11, *P* = .001). Subsequently, these three genes were summed and divided in tertiles (low, intermediate and high expression). As expected, higher transcript levels of these three genes were strongly associated with poorer OS and EFS in the cohort of 218 cytogenetically normal karyotype AMLs (*P* = .007 and *P* = .006, respectively). Also in the independent cohort of 163 normal karyotype AMLs Kaplan-Meier curves show that patients with high expression levels of these three genes have a significantly worse OS compared to patients with low expression levels (*P* < .001).

### Prognostic value of ANKRD28, GNA15 and UGP2 expression level in the context of known disease-related risk factors

Well known disease-related risk factors such as *FLT3*-ITD, *NPM1c*⁺ and *CEBPA* mutation status were considered in a multivariable analysis in the cohort of 218 cytogenetically normal karyotype AMLs for both OS (*ANKRD28*: HR: 1.32, 95% CI: 1.07-1.62, *P* = .008; *GNA15*: HR: 1.22, 95% CI: 1.02-1.47, *P* =.033 and *UGP2:* HR: 1.86, 95% CI: 1.17-2.97, *P* = .009) as well as EFS (*ANKRD28:* HR: 1.31, 95% CI: 1.09-1.59, *P* = .005; *GNA15:* HR: 1.21, 95% CI: 1.02-1.43, *P* = .030 and *UGP2:* HR: 1.73, 95% CI: 1.11-2.68, P=.015) (details in Table 3). These data indicate that the continuous transcript levels of *ANKRD28, GNA15* and *UGP2* are independent risk indicators for both OS and EFS.

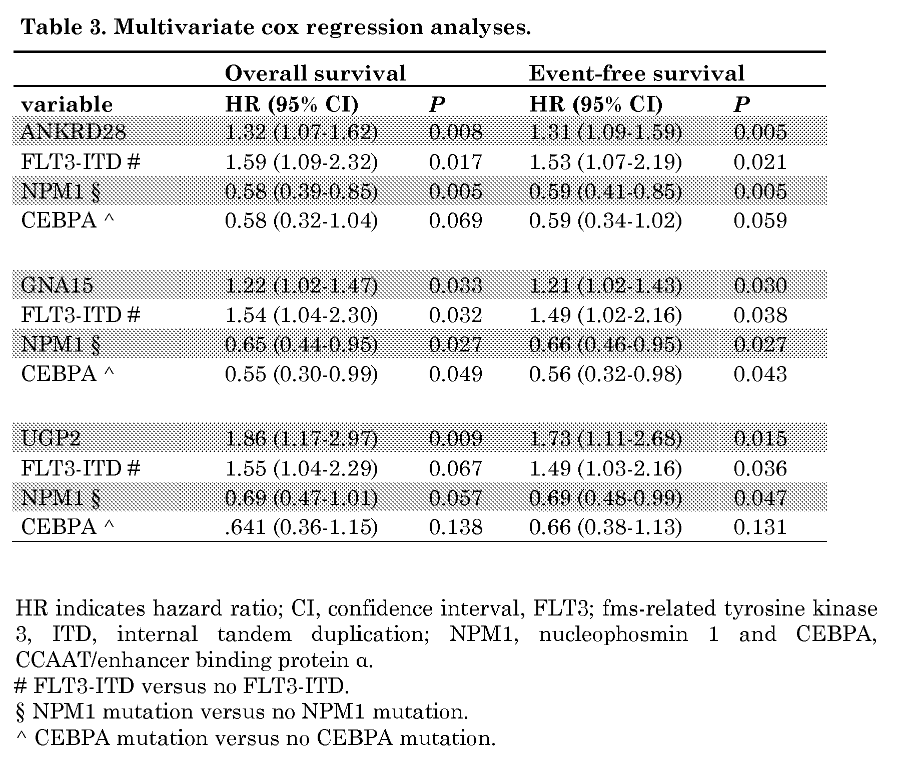

### REFERENCES

1. Vardiman JW, Thiele J, Arber DA et al. Blood 2009;114:937-951.
2. Falini B, Mecucci C, Tiacci E et al. N.Engl.J.Med. 2005;352:254-266.
3. Nakao M, Yokota S, Iwai T et al. Leukemia 1996;10:1911-1918.
4. Wouters BJ, Lowenberg B, Erpelinck-Verschueren CA et al. Blood 2009;113:3088-3091.
5. Valk PJ, Verhaak RG, Beijen MA et al. N.Engl.J.Med. 2004;350:1617-1628.
6. Bullinger L, Dohner K, Bair E et al. N.Engl.J.Med. 2004;350:1605-1616.
7. Alcalay M, Tiacci E, Bergomas R et al. Blood 2005;106:899-902.
8. Radmacher MD, Marcucci G, Ruppert AS et al. Blood 2006;108:1677-1683.
9. Wouters BJ, Lowenberg B, Delwel R. Blood 2009;113:291-298.
10. Wouters BJ, Lowenberg B, Erpelinck-Verschueren CA et al. Blood 2009; 113:3088-3091.
11. Bonnet D, Dick JE. Nat.Med. 1997;3:730-737.
12. Lapidot T, Sirard C, Vormoor J et al. Nature 1994;367:645-648.
13. Warner JK, Wang JC, Hope KJ, Jin L, Dick JE. Oncogene 2004;23:7164-7177.
14. Wang JC, Dick JE. Cancer stem cells: lessons from leukemia. Trends Cell Biol. 2005;15:494-501.
15. Blair A, Hogge DE, Ailles LE, Lansdorp PM, Sutherland HJ. Blood 1997;89:3104-3112.
16. Blair A, Hogge DE, Sutherland HJ. Blood 1998;92:4325-4335.
17. Blair A, Sutherland HJ. Exp.Hematol. 2000;28:660-671.
18. Hosen N, Park CY, Tatsumi N et al. Proc.Natl.Acad.Sci.U.S.A 2007;104:11008-11013.
19. Jordan CT, Upchurch D, Szilvassy SJ et al. Leukemia 2000;14:1777-1784.
20. Taussig DC, Pearce DJ, Simpson C et al. Blood 2005;106:4086-4092.
21. Taussig DC, Miraki-Moud F, Anjos-Afonso F et al. Blood 2008;112:568-575.
22. Cornelissen JJ, van Putten WL, Verdonck LF et al. Blood 2007;109:3658-3666.
23. van der Holt B, Breems DA, Berna Beverloo H et al. Br.J.Haematol. 2007;136:96-105.
24. Yokota S, Kiyoi H, Nakao M et al. Leukemia 1997;11:1605-1609.
25. Irizarry RA, Bolstad BM, Collin F et al. Nucleic Acids Res. 2003;31:e15.
26. Bolstad BM, Irizarry RA, Astrand M, Speed TP. Bioinformatics. 2003;19:185-193.
27. Alter O, Brown PO, Botstein D. Proc.Natl.Acad.Sci.U.S.A 2000;97:10101-10106.
28. Crijns AP, Fehrmann RS, de Jong S et al. PLoS.Med. 2009;6:e24.
29. Crijns AP, Gerbens F, Plantinga AE et al. BMC.Genomics 2006;7:232.
30. Sherlock G. Brief.Bioinform. 2001;2:350-362.
31. de Jonge HJ, de Bont ES, Valk PJ et al. Blood 2009;114:2869-2877.
32. Metzeler KH, Hummel M, Bloomfield CD et al. Blood 2008;112:4193-4201.
33. Eisen MB, Spellman PT, Brown PO, Botstein D. Proc.Natl.Acad.Sci.U.S.A 1998;95:14863-14868.

## Claims

1. A method for predicting the disease prognosis of a subject suffering from acute myeloid leukemia (AML), said method comprising:
a. obtaining an isolated sample from a subject;
b. screening for a simultaneous aberrant expression level of two or more biomarkers in the sample, the biomarkers being selected from the group consisting of ankyrin repeat domain 28 (ANKRD28), guanine nucleotide binding protein alpha 15 (GNA15), UDP-glucose pyrophosphorylase (UGP2) and
c. scoring the expression level as being aberrant when the expression level detected is above a certain threshold coefficient; wherein the detection threshold coefficient is determined by comparing the expression levels of the samples obtained from the subject to values in a reference database of sample phenotypes obtained from healthy subjects, wherein the presence of an aberrant expression level of two or more markers is indicative of a poor disease prognosis in the subject.

2. Method according to claim 1, wherein the aberrant expression level is an increased cumulative expression of two or more biomarkers in the sample, the biomarkers being selected from the group consisting of ankyrin repeat domain 28 (ANKRD28), guanine nucleotide binding protein alpha 15 (GNA15), UDP-glucose pyrophosphorylase (UGP2).

3. Method according to claim 1 or 2, comprising the screening for a simultaneous increased expression level of ANKRD28, GNA15 and UGP2.

4. Method according to any one of claims 1-3, wherein the subject suffers from normal karyotype (NK) AML.

5. Method according to any one of the preceding claims, wherein the poor disease prognosis is selected from the group consisting of recurrence, relapse, poor overall survival prognosis, poor disease free survival, or a combination thereof.

6. A predictor set suitable for use in a method according to any one of claims 1-5, comprising a collection of two or more isolated binding partners which bind selectively to the products of at least two biomarkers, wherein the biomarkers are selected from the group consisting of ankyrin repeat domain 28 (ANKRD28), guanine nucleotide binding protein alpha 15 (GNA15) and UDP-glucose pyrophosphorylase (UGP2).

7. Predictor set according to claim 6, consisting of a collection of distinct isolated binding partners which bind selectively to the products of the biomarkers ANKRD28, GNA15 and UGP2.

8. Predictor set according to claim 6 or 7, wherein the binding partners and the biomarker products are proteins.

9. Predictor set according to claim 8, wherein the binding partners are antibodies, preferably monoclonal antibodies.

10. Predictor set according to claim 6, comprising a collection of two or more isolated polynucleotides each of which binds selectively to the RNA products of at least two biomarkers.

11. Predictor set according to claim 10, wherein the isolated polynucleotides are single or double stranded RNA or single or double stranded DNA.

12. Predictor set according to any one of claims 6 to 11, the binding partners being bound to a solid support, preferably wherein said binding partners are bound to said solid support in an addressable location

13. A diagnostic kit comprising a predictor set according to any one of claims 6 to 12, and instructions for use to predict the disease prognosis of a subject suffering from AML, preferably NK-AML.

14. A method for choosing the treatment modality for an individual subject suffering from AML, comprising predicting the disease prognosis of said subject according to a method of any one of claims 1-5, using a predictor set according to any one of claims 6-12, and/or using a kit according to claim 13, and further comprising selecting a treatment regimen based on the disease prognosis, preferably wherein selecting a treatment regimen comprises determining whether the therapy includes or excludes a bone marrow transplantation.

15. A method for identifying a biomarker useful for predicting the disease prognosis of an AML subject, comprising the steps of:
a. providing a first population of mononuclear cells derived from subjects known to suffer from AML
b. enriching said population for CD34 positive mononuclear cells
c. performing gene expression profiling on said enriched population
d. comparing the gene expression profile with a profile obtained from CD34 enriched population of healthy subjects
e. determining which genes are differentially expressed between the AML
f. performing univariate Cox regression analyses between the differentially expressed genes and disease progression in a population of de novo cytogenetically normal AMS
g. identifying the gene(s) showing a significant (P<0.05) association with poor disease progression.
